# EUROPÄISCHE PATENTANMELDUNG

(11) **EP 4 796 146 A2**
(43) Veröffentlichungstag der Anmeldung: **26.08.2026**
(21) Anmeldenummer: 26185817.9
(22) Anmeldetag: 11.11.2022
(51) Int. Cl.: A61M 5/315

(54) **MEHRKOMPONENTIGER STOPFEN FÜR EINE MEDIZINISCHE SPRITZE SOWIE MEDIZINISCHE SPRITZE**

(62) Dokumentnummer(n) der früheren Anmeldung(en) nach Art. 76 EPÜ: 22206993.2 / 4 368 224
(71) Anmelder: Inductio AG, 6052 Hergiswil (CH)
(72) Erfinder: Spichiger, Marco, 2544 Bettlach (CH); Koller, Horst, 8730 Uznach (CH)
(74) Vertreter: Tergau & Walkenhorst Intellectual Property GmbH

(57) **Zusammenfassung**

Die Erfindung betrifft eine mehrkomponentig ausgeführte Nadelschutzkappe (12, 12', 12'') für eine medizinische Spritze (1), mit einem Außenkörper (100) aus einem ersten, vergleichsweise harten Kunststoff, der innenseitig mit einer Auskleidung (102) aus einem zweiten, im Vergleich zum ersten Kunststoff weicheren Kunststoff versehen ist.

## Beschreibung

Die Erfindung bezieht sich auf eine Nadelschutzkappe für eine medizinische Spritze. Sie betrifft weiter eine medizinische Spritze mit einer solchen Nadelschutzkappe.

Im Spritzenkörper einer medizinischen Spritze ist üblicherweise ein in Längsrichtung verschiebbarer Kolben oder Stopfen vorgesehen, der über ein am proximalen Ende des Spritzenkörpers angeordnetes Betätigungselement, beispielsweise einen Betätigungsstößel oder auch ein Element mit automatisiertem Antrieb, verschoben werden kann. Damit kann über einen solchen Stopfen Druck auf den im Spritzenkörper vorgehaltenen Wirkstoff ausgeübt werden, so dass dieser über eine am distalen Ende des Spritzenkörpers angeordnete Hohlnadel ausgetragen und somit appliziert werden kann.

Um vor dem Gebrauch einer solchen Spritze ein unbeabsichtigtes Stechen zu vermeiden, und auch um das in der Spritze vorgehaltene Mittel oder den Wirkstoff sicher zu verwahren, kann eine solche Spritze mit einer Nadelschutzkappe versehen sein. Diese umgibt üblicherweise die am distalen Ende des Spritzenkörpers angebrachte Hohlnadel vollständig, und sie wird unmittelbar vor Gebrauch der Spritze vom Spritzenkörper abgezogen, um die Hohlnadel freizulegen und die Spritze somit einsatzfertig zu machen.

Der Erfindung liegt nunmehr die Aufgabe zugrunde, eine besonders verbesserte Nadelschutzkappe der genannten Art anzugeben.

Diese Aufgabe wird erfindungsgemäß gelöst mit einer Nadelschutzkappe mit einem Außenkörper aus einem ersten, vergleichsweise harten Kunststoff, der innenseitig mit einer Auskleidung aus einem zweiten, im Vergleich zum ersten Kunststoff weicheren Kunststoff versehen ist.

Vorteilhafte Ausgestaltungen der Erfindung sind in den Unteransprüchen angegeben und ergeben sich durch Merkmale und Aspekte gemäß nachfolgender Beschreibung.

Die Erfindung geht von der Überlegung aus, dass eine hinsichtlich ihrer Eigenschaften verbesserte Nadelschutzkappe einerseits für eine besondere Stabilität im Sinne einer sicheren Verwahrung der umschlossenen Hohlnadel, andererseits aber auch für eine besonders zuverlässige Abdichtwirkung der Hohlnadel ausgelegt sein sollte. Um dies zu ermöglichen, ist gemäß einem .Aspekt der Erfindung die Nadelschutzkappe mehr-, insbesondere zwei-, komponentig ausgebildet, so dass einerseits über die erste Komponente, nämlich einen vergleichsweise stablien Außenkörper aus vergleichsweise hartem Kunststoff, die gewünschte Stabilität und mechanische Absicherung der Hohlnadel, und andererseits über die zweite Komponente, nämlich einer innenseitig im Außenkörper angebrachten Auskleidung, die gewünschte Abdichtung der Hohlnadel gezielt bereitgestellt werden kann.

In vorteilhafter Weiterbildung ist die zweikomponentige Nadelschutzhülle durch ein 2K-Spritzgussverfahren hergestellt. Der Außenkörper kann dabei bevorzugt aus PP gebildet sein; in zusätzlicher oder alternativer vorteilhafter Ausgestaltung ist zudem die Auskleidung aus TPE ausgeführt.

Aspekte der Erfindung umfassen zudem eine medizinische Spritze mit einem Spritzenkörper, an dessen distalem Ende eine in einem Nadelkopf angeordnete Hohlnadel angeordnet ist, und mit einer Nadelschutzkappe der vorstehend beschriebene Art.

Gemäß einem vorteilhaften Aspekt umfasst die Spritze dabei eine Auskleidung der Nadelschutzkappe, die in deren frontalem Endbereich derart verdickt ausgeführt und positioniert ist, dass bei auf den Spritzenkörper aufgesetzter Nadelschutzkappe die Hohlnadel mit ihrer Spitze in den frontalen Endbereich der Auskleidung eindringt und somit dort fixiert und abgedichtet wird.

In einem weiteren vorteilhaften Aspekt der Erfindung ist die Auskleidung der Nadelschutzkappe in einem zur Aufnahme des vorderen Bereichs des Nadelkopfs vorgesehenen Aufnahmebereich mit ihrer Innenkontur an die Außenkontur des Nadelkopfs angepasst.

In einem weiteren, als unabhängig erfinderisch oder insbesondere auch in Kombination mit den vorstehend beschriebenen Aspekten als erfinderisch angesehenen Aspekt umfasst die Erfindung zudem einen mehrkomponentigen Stopfen mit einem zentralen Stopfenkörper aus einem ersten, vergleichsweise harten Kunststoff, und mit einem den zentralen Stopfenkörper zumindest teilweise umschließenden Dichtmantel aus einem zweiten, im Vergleich zum ersten Kunststoff weicheren Kunststoff, wobei der Dichtmantel einen im Wesentlichen zylindrischen Außenmantel aufweist, der mit einer Mehrzahl von in Richtung der Zylinderachse gesehen beabstandet zueinander positionierten umlaufenden Dichtrippen versehen ist.

Dabei kann berücksichtigt sein, dass ein hinsichtlich seiner Eigenschaften verbesserter Stopfen zusätzlich zu einer hohen Abdichtwirkung auch besonders gute Gleit- und Reibeieigenschaften bei einer Verschiebung im Spritzenkörper aufweisen sollte. Dabei sollte insbesondere die Haft- oder Gleitreibung des Stopfens an der Innenwand des Spritzenkörpers besonders gering gehalten werden, um eine vergleichsweise sanfte und schonende Bewegung innerhalb des Spritzenkörpers mit gering gehaltenen Losbrechkräften und dergleichen zu ermöglichen. Gemäß einem Aspekt der vorliegenden Erfindung kann dies erreicht werden durch eine gezielte Nutzung der Verformbarkeit des Dichtmantels aufgrund seiner Materialeigenschaften eines im Vergleich zum zentralen Stopfenkörper weicheren Materials. Diese kann mittels einer geeigneten Formgebung des Dichtmantels besonders wirksam genutzt werden, wobei durch ein geeignetes Außenprofil geeignete Verformungszonen bereitgestellt werden können.

Insbesondere erlaubt die gemäß einem Aspekt der Erfindung vorgesehene Bauweise, also die Bereitstellung des inneren, vergleichsweise härteren Bauteils des Kolbenstopfens, darin ein stabiles Gewinde zu formen, welches mit dem Gegenstück (Betätigungselement, z. B. Betätigungsstössel oder auch Kolbenstange) eine feste Verbindung bilden kann. Durch eine solche Gewindeverbindung zwischen Betätigungselement, insbesondere Betätigungsstößel, und Stopfen können axiale Verschiebungen des Stopfens sowohl in "Vorwärts- " als auch in "Rückwärtsrichtung" erfolgen, d. h. es können wahl- und bedarfsweise Schub- oder auch Zugwirkungen auf den Stopfen ausgeübt werden. Damit sind auch Anwendungen wie z. B. Aspirationstests und/oder Rekonstitutionen von Medikamenten in einer solchen Spritze ermöglicht.

Vorteilhafterweise ist zu diesem Zweck die Breite der oder jeder Dichtrippe des Dichtmantels geringer gewählt als der Abstand zwischen zwei benachbarten Dichtrippen. In alternativer oder zusätzlicher vorteilhafter Weiterbildung weist der Dichtmantel in seinem endseitigen Bereich einen im Vergleich zum Außendurchmesser des zentralen Stopfenkörpers vergrößerten Innendurchmesser auf, so dass sich im montierten Zustand eine endseitig umlaufende Nut zwischen dem zentralen Stopfenkörper und dem Dichtmantel ergibt. Sowohl diese Nut als auch die Raumbereich zwischen benachbarten Dichtrippen können dann als Verformungszonen für die Dichtlippen selbst dienen, so dass deren Anpresskraft an die Innenwand des Spritzenkörpers geringgehalten werden kann.

Bevorzugt und in als eigenständig erfinderisch angesehener Ausgestaltung ist eine medizinische Spritze mit einem Spritzenkörper ausgestattet, in dem ein mehrkomponentiger Stopfen der genannten Art geführt ist.

Ein Ausführungsbeispiel der Erfindung wird anhand einer Zeichnung näher erläutert. Darin zeigen:
- FIG. 1: eine medizinische Spritze in perspektivischer Ansicht,
- FIG. 2: die Spritze nach FIG. 1 mit entfernter Nadelschutzkappe in perspektivischer Ansicht,
- FIG. 3: den Nadelkopf der Spritze gemäß FIG. 1 in perspektivischer Ansicht,
- FIG. 4: den Nadelkopf der Spritze gemäß FIG. 1 in seitlicher Ansicht,
- FIG. 5: den Nadelkopf der Spritze gemäß FIG. 1 in einem Zwischenzustand in seitlicher Ansicht,
- FIG. 6: den Nadelkopf der Spritze gemäß FIG. 1 in einsatzbereitem Zustand in seitlicher Ansicht,
- FIG. 7: den Nadelkopf der Spritze gem. FIG. 1 in entsorgungsbereitem Zustand,
- FIG. 8: einen Nadelhalter der Spritze gem. FIG. 1 in perspektivischer Ansicht,
- FIG. 9: den Nadelhalter gem. FIG. 8 im Längsschnitt,
- FIG. 10: den Nadelhalter gem. FIG. 8 mit aufgesteckter Nadelschutzkappe in perspektivischer Ansicht,
- FIG. 11: einen Spritzenkörper mit aufgeschraubtem Ensemble gem. FIG. 10 im Längsschnitt,
- FIG. 12: einen alternativen Nadelhalter der Spritze gem. FIG. 1 in seitlicher Ansicht,
- FIG. 13: den Nadelhalter gem. FIG. 12 im Längsschnitt,
- FIG. 14: den Nadelhalter gem. FIG. 12 mit aufgesteckter Nadelschutzkappe in seitlicher Ansicht,
- FIG. 15: einen Spritzenkörper mit vorläufig aufgestecktem Ensemble gem. FIG. 14 im Längsschnitt,
- FIG. 16: einen Spritzenkörper mit aufgestecktem Ensemble gem. FIG. 14 im Längsschnitt,
- FIG. 17: eine Nadelschutzkappe der Spritze nach FIG. 1 im Längsschnitt,
- FIG. 18: einen im Spritzenkörper nach FIG. 11 bzw. nach FIG. 17 geführten Stopfen im Längsschnitt,
- FIG. 19: den Stopfen nach FIG. 18 in perspektivischer Ansicht,
- FIG. 20: den Stopfen nach FIG. 18 in seitlicher Ansicht,
- FIG. 21: den Stopfen nach FIG. 18 in perspektivischer Ansicht,
- FIG. 22: einen alternativen Spritzenkörper im Längsschnitt,
- FIG. 23: eine Nadelschutzkappe des Spritzenkörpers gem. FIG. 22 im Längsschnitt,
- FIG. 24: die Nadelschutzkappe gem. FIG. 23 in perspektivischer Ansicht, und
- FIG. 25: die Nadelschutzkappe gem. FIG. 23 in seitlicher Ansicht.

Gleiche Teile sind in allen Figuren mit denselben Bezugszeichen versehen.

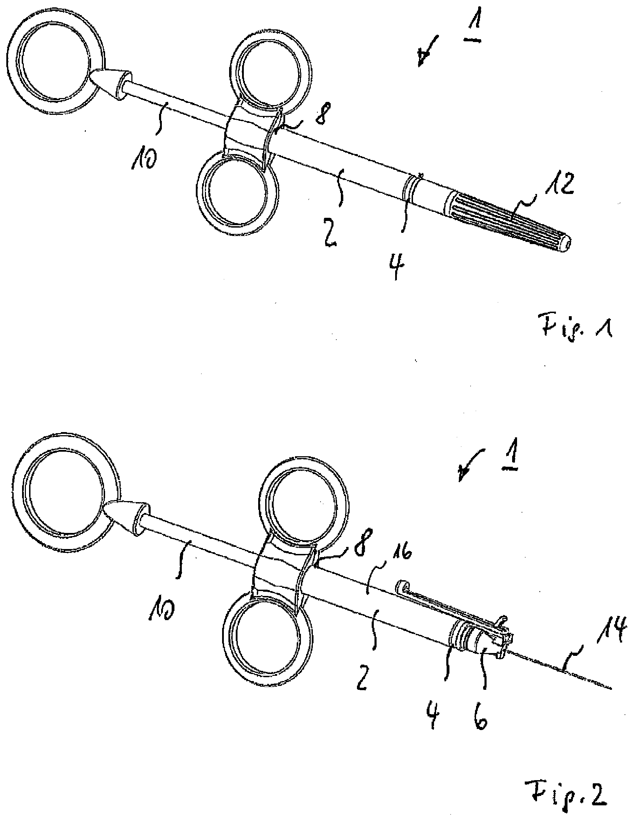

Die medizinische Spritze 1 gemäß FIG. 1 umfasst im Wesentlichen drei Baugruppen, nämlich einen den Spritzenkörper 2 bildenden Wirkstoffbehälter (auch als Kartuschen- oder Patroneneinheit bezeichnet), der einerseits an seinem distalen Ende 4 mit einem Nadelkopf 6 oder Nadelhalter und andererseits an seinem proximalen Ende 8 mit einem Betätigungselement 10 verbunden ist. In FIG. 1 ist die Spritze 1 zudem mit am Nadelkopf 6 angebrachter Nadelschutzkappe 12 gezeigt. FIG. 2 zeigt die Spritze 1 hingegen in einsatzfähigem Zustand mit entfernter Nadelschutzkappe 12, so dass die im Nadelkopf 6 angebrachte Hohlnadel 14 freiliegt.

Im Ausführungsbeispiel ist als Betätigungselement 10 ein Griffstück gezeigt, wie es beispielsweise in einer zahnärztlichen Behandlung bei der Anästhesie zum Einsatz kommen könnte. Es umfasst im gezeigten Beispiel ein mit Fingerringen versehenes Basiselement, in dem ein endseitig ebenfalls mit einer Fingerlasche versehener Betätigungsstößel in seiner Längsrichtung verschiebbar geführt ist. Alternativ kann das Betätigungselement 10 aber auch als gewöhnliches Spritzen-Griffstück oder aber auch als automatisiertes Betätigungselement 10 in der Art eines Autoinjektors ausgeführt sein. Die Verbindung des Betätigungselements 10 mit dem Spritzenkörper 2 kann in unterschiedlichen Variationen ausgeführt sein, beispielsweise als Schraub-, Steck- oder Klickverbindung.

Der Spritzenkörper 2 ist entsprechend einer herkömmlichen Bauweise zylindrisch oder rohrförmig als ein Spritzengehäuse bildender Hohlkörper 16 ausgeführt, der zur Aufnahme des medizinischen Wirkstoffs vorgesehen ist. Der an den Spritzenkörper 2 anbringbare Nadelkopf 6, der im Moment der Anbringung noch mit der Nadelschutzkappe 12 versehen ist, weist dabei eine an sich als eigenständig erfinderisch angesehene Ausgestaltung auf. Dabei ist dem Umstand Rechnung getragen, dass es ein zunehmendes Bedürfnis und teilweise auch schon in rechtliche oder regulatorische Vorschriften eingeflossen ist, medizinische Spritzen mit so genannten Nadelschutzsystemen auszurüsten. Zunehmende gesetzliche Vorgaben zur sicheren Arbeitsumgebung in verschiedenen Ländern verpflichten nämlich beispielsweise die Arbeitgeber im medizinischen Bereich zum Schutz ihrer Mitarbeit er vor Nadelstichverletzungen. Dazu kann dann vorgesehen sein, beispielsweise ein System aus Schutzkappen vorzusehen, die über die Nadel gesteckt werden, wenn diese nicht in Benutzung ist, oder es können so genannten Retraktionssysteme vorgesehen sein, bei denen die Nadel nach Gebrauch in das zwischenzeitlich vom Wirkstoff entleerte Spritzengehäuse zurückgezogen wird, so dass die Nadelspitze nicht mehr freiliegt.

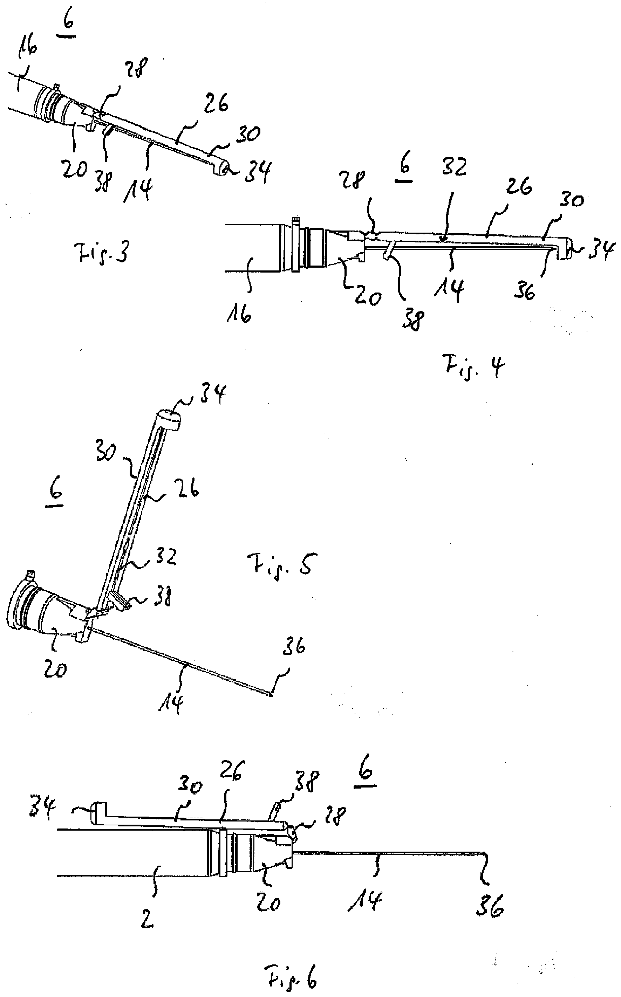

Bei der vorliegenden, als eigenständig erfinderisch angesehenen Ausführungsform ist der an den Hohlkörper 16 anbringbare Nadelkopf 6 oder Nadelhalter mit einem integrierten Nadelschutzsystem versehen. Der Nadelkopf 6 - nach Entfernung der Nadelschutzkappe 12 - ist in perspektivischer Ansicht in FIG. 3 und in seitlicher Ansicht in FIG. 4 jeweils vergrößert gezeigt.

Wie diesen Darstellungen gut entnehmbar ist, umfasst der Nadelkopf 6 einen den eigentlichen Nadelhalter bildenden Grundkörper 20, vorzugsweise gefertigt aus einem harten Kunststoff wie beispielsweise PP, POM oder PC. Der Grundkörper 20 lagert und trägt dabei die Hohlnadel 14.

Zur Bereitstellung des gewünschten Nadelschutzes ist am Grundkörper 20 eine Nadelschutzhülle 26 über ein Gelenk 28 schwenkbar angebracht. Die Nadelschutzhülle 26 wird dabei von einem länglich ausgedehnten Schutzkörper 30 gebildet, der in seiner Länge die freie Länge der Hohlnadel 14 übertrifft und diese somit endseitig überragt. Des Weiteren ist die Breite des Schutzkörpers 30 derart gewählt, dass er die Hohlnadel 14 beidseitig nach außen hin deutlich überragt. Der Schutzkörper 30 kann zudem innenseitig eine Aufnahmerinne 32 aufweisen, in die die Hohlnadel 14 in der in den FIG. 3, 4 gezeigten Position des Schutzkörpers 30 eingebettet sein kann. Endseitig ist am Schutzkörper 30 eine Deckplatte 34 angeformt, die in der dort gezeigten Position das freie Ende 36 der Hohlnadel 14 abdeckt. In der in den FIG. 3, 4 gezeigten Position ist das freie Ende 36 der Hohlnadel 14 somit vollständig abgeschirmt, und kein Kontakt und somit auch keine Verletzung durch die Hohlnadel 14 ist damit möglich.

In dieser Position befindet sich das System, wenn die äußere Schutzkappe 12 abgenommen wird. Da die Hohlnadel 14 in diesem Zustand noch von dem Schutzkörper 30 abgeschirmt wird, ist noch kein Einstechen der Nadel 14 möglich. Um diese einsetzbar zu machen, wird daher mittels eines am Schutzkörper 30 angeformten Betätigungselements 38 um das Gelenk 28 herum von der Hohlnadel 14 weggeschwenkt, so dass diese nunmehr freigelegt und freigegeben wird. Zur Verdeutlichung zeigen FIG. 5 eine Zwischenposition mit halb abgeschwenktem Schutzkörper 30 und FIG. 6 eine Endposition, in der der Schutzkörper 30 vollständig von der Nadel 14 abgeschwenkt ist und rückseitig an der Außenseite des Hohlkörpers 16 anliegt. In diesem Zustand ist die Spritze 1 nun zur Abgabe des Wirkstoffs bereit.

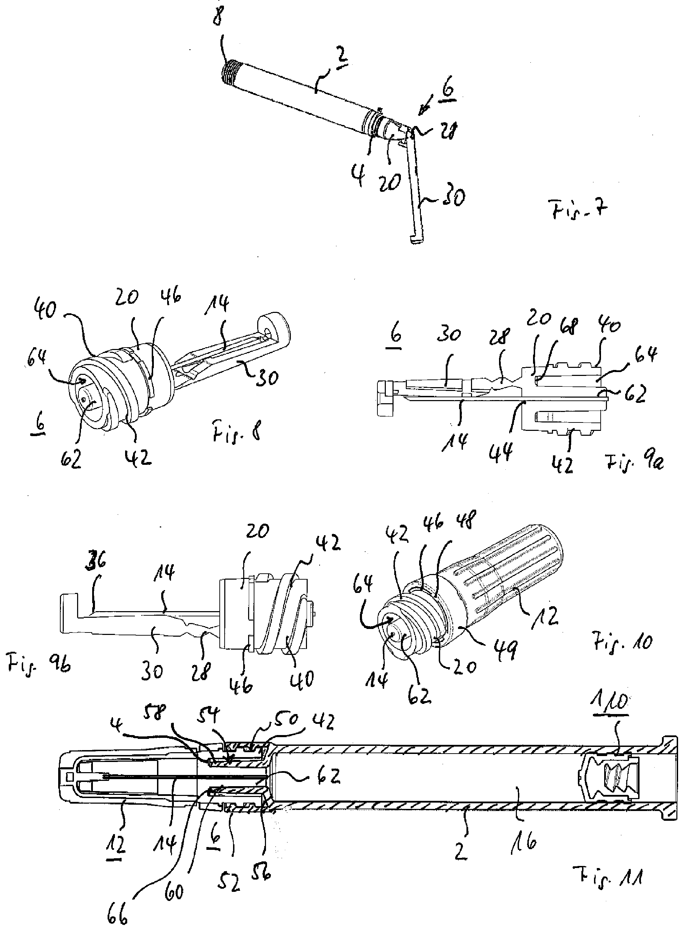

Nach Gebrauch der Spritze 1 kann der Schutzkörper 30 um das Gelenk 28 herum wieder zurück geschwenkt werden, so dass er die Hohlnadel 14 erneut wie vorstehend beschrieben abschirmt und damit Verletzungen durch Berührungen der Nadel 14 sicher vermieden sind. Um aber die Verletzungsgefahr an der dann benutzten Nadel 14 noch weiter zu vermindern, ist gemäß einem weiteren Aspekt der Erfindung vorgesehen, den Schutzkörper 30 um das Gelenk 28 herum noch weiter zu verschwenken, bis die Nadel 14 in ihrem Nadellager im Grundkörper 20 abbricht. Damit wird, wie in FIG. 7 dargestellt, die Nadel 14 nunmehr endgültig in ihrer Position innerhalb des Abschirmbereichs des Schutzkörpers 30 fixiert, und zudem wird damit unmittelbar deutlich, dass die Spritze 1 bereits benutzt war und der (nunmehr verbrauchte) Spritzenkörper 2 gemeinsam mit der noch daran montierten, nunmehr zerbrochenen Nadel 14 entsorgt werden soll. Damit sind Irrtümer und Verwechslungen, die beispielsweise zu einem versehentlichen Wiederbenutzen der Spritze 1 führen könnten, sicher ausgeschlossen.

Der Nadelkopf 6 oder Nadelhalter kann am vorderen oder distalen Ende 4 des Hohlkörpers 16 angeschraubt oder auch aufgesteckt werden, je nachdem, welche Bauweise für die Verbindung gewählt ist. In einer ersten, aufschraubbaren Ausführung ist der Nadelkopf 6 in FIG. 8 in perspektivischer Ansicht und in FIG. 9 im Längsschnitt bzw. in seitlicher Ansicht (FIG. 9b) gezeigt. In dieser Variante ist der Grundkörper 20 gemäß einem Aspekt der Erfindung in einem Anschlussbereich 40 außenseitig mit einem Luer-Gewinde 42 versehen, das in ein angepasstes Luer-Innengewinde am distalen Ende 4 des Spritzenkörpers 2 eingeschraubt werden kann. Die zur verbesserten Verbindung mit dem Grundkörper 20 umspritzt ausgeführte Hohlnadel 14 ist dabei in einem innerhalb des Grundkörpers 20 vorgesehenen Nadellager 44 geführt. Oberhalb des Luer-Gewindes 42 ist der Grundkörper 20 außenseitig mit einer umlaufenden, segmentweise durchbrochenen Rippe 46 versehen. Auf den Grundkörper 20 des Nadelhalters 6 kann gemäß einem Aspekt der Erfindung in der Art einer Vormontage die Nadelschutzkappe 12 aufgesteckt werden, wobei eine Anzahl von innenseitig an der Nadelschutzkappe 12 angeordnete Rastnasen 48 jeweils in eines der ausgesparten Segmente der Rippe 46 eingreifen. Der Grundkörper 20 und die aufgesteckte Nadelschutzkappe 12 bilden somit ein rotatorisches Gesperre mit Formschluss in Rotationsrichtung, so dass das in FIG. 10 gezeigte Ensemble aus Nadelhalter 6 und aufgesteckter Nadelschutzkappe 12 gemeinsam auf den Spritzenkörper 2 aufgeschraubt werden kann. Dabei wird die Nadelschutzkappe 12 gedreht, und die der mit dem Gewinde 42 versehene Grundkörper 20 wird durch das Gesperre in Rotationsrichtung mitgenommen, so dass das Gewinde 42 aufgeschraubt wird.

Wie der Darstellung in FIG. 10 zudem entnehmbar ist, ist gemäß einem Aspekt der Erfindung die Nadelschutzkappe 12 als Originalitätsverschluss ausgeführt. Dazu umfasst sie den eigentlichen Kappenkörper, an den in der Art eines Siegels abreißbar ein Sicherungsring 49 angeformt ist. Beim Öffnen der Spritze, also beim Entfernen der Nadelschutzkappe 12 vom Nadelhalter 6, wird der Sicherungsring 49 abgerissen, und er verbleibt auf dem Nadelhalter 6. Damit wird für den Benutzer eindeutig erkennbar, dass die Spritze 1 bereits benutzt und der Wirkstoffcontainer "angebrochen" wurde.

Der mit der aufgeschraubten Nadelschutzkappe 12 versehene Spritzenkörper 2 ist in FIG. 11 im Längsschnitt gezeigt. Deutlich ist dabei erkennbar, dass der in dieser Variante an die Konfiguration des Nadelhalters 6 angepasste Spritzenkörper 2 an seinem distalen Ende 4 mit dem Luer-Innengewinde 50 versehen ist. Dieses ist innenseitig an einen endseitigen Anschlussstutzen 52 des Spritzenkörpers 2 angeformt. Der Anschlussstutzen 52 umgibt dabei in seinem Innenbereich ein Verbindungssystem 54 für den Nadelhalter 6. Das Verbindungssystem 54 umfasst gemäß einem Aspekt der Erfindung einen unter Freilassung eines umlaufenden Gewinderaums 56 zentral angeordneten Verbindungszapfen 58, der seinerseits mittig einen sich zum distalen Ende 4 hin aufweitenden Aufnahmekanal 60 für einen zugeordneten, geometrisch an diesen angepassten, das Nadellager 44 tragenden Steckzapfen 62 des Nadelhalters 6 aufweist. Beim Einschrauben des Luer-Gewindes 42 in das daran angepasste Luer-Innengewinde 50 des Spritzenkörpers 2 wird somit der Steckzapfen 62 zunehmend in den Aufnahmekanal 60 eingebracht, bis aufgrund der konischen Ausgestaltung des Steckzapfens 62 einerseits und daran angepasst des Aufnahmekanals 60 andererseits ein flächiger Kontakt zwischen diesen Komponenten entsteht. Damit ist gemäß einem Aspekt der Erfindung eine besonders dichte und formbedingt auch mechanisch besonders stabile Verbindung des Nadelhalters 6 mit dem Spritzenkörper 2 erreicht.

Der Grundkörper 20 bildet in seinem endseitigen Anschlussbereich 40 somit eine in Längsrichtung ausgerichtete ringförmige Vertiefung 64 aus, in die der den Aufnahmekanal 60 ringartig umschließende Kragen 66 des Verbindungszapfens 58 eingebracht werden kann. In ihrem Bodenbereich ist diese Vertiefung 64 gemäß einem Aspekt der Erfindung mit einer Anzahl von Rastnocken 68 versehen, in die korrespondierende Nocken an der Stirnfläche des Kragens 66 einschnappen können, sobald das Luer-Gewinde 42 vollständig in das Luer-Innengewinde 50 eingeschraubt wurde. Damit kann die Luer-Verbindung zwischen Spritzenkörper 2 und Nadelhalter 6 nach dem vollständigen Einschrauben rotationsgesichert und somit fixiert werden.

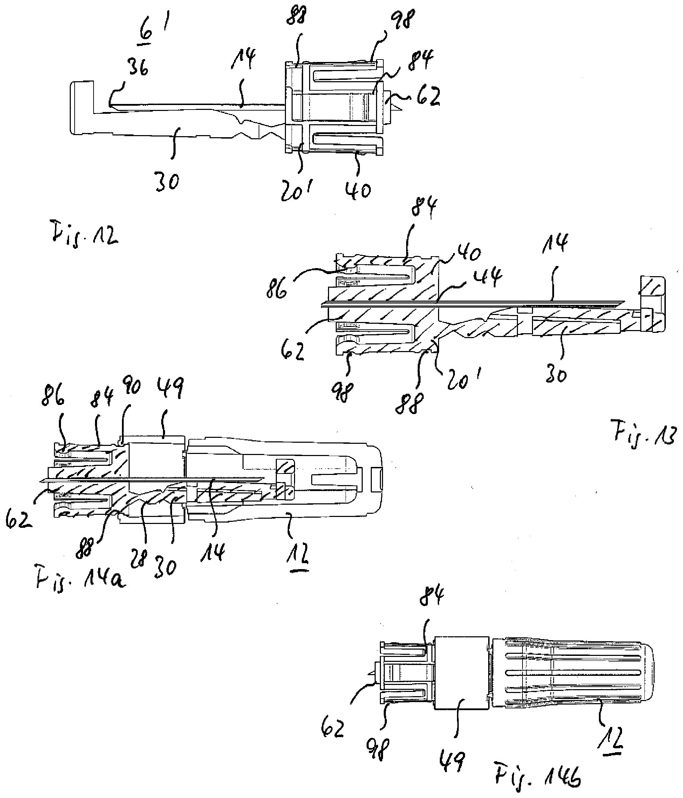

In einer zweiten, als eigenständig erfinderisch angesehenen aufsteckbaren Ausführung ist der Nadelkopf 6´ in FIG. 12 in seitlicher Ansicht und in FIG. 13 im Längsschnitt gezeigt. In dieser Variante ist der Grundkörper 20´ gemäß einem Aspekt der Erfindung in seinem Anschlussbereich 40 als Aufprellkappe 84 ausgeführt, an deren Innenumfang eine Anzahl von mit einer zugeordneten Außenwulst im proximalen Endbereich des Spritzenkörpers 2´ in Eingriff bringbaren Schnapphaken oder Rastelementen 86 angeordnet ist. Die auch in diesem Ausführungsbeispiel zur verbesserten Verbindung mit dem Grundkörper 20´ umspritzt ausgeführte Hohlnadel 14 ist dabei ebenfalls in einem innerhalb des Grundkörpers 20´ vorgesehenen Nadellager 44 geführt. Am oberen Rand der Aufprellkappe 84 ist der Grundkörper 20´ außenseitig mit einer umlaufenden Nut 88 versehen. Auf den Grundkörper 20´ des Nadelkopfs 6´ kann gemäß einem Aspekt der Erfindung auch in dieser Variante in der Art einer Vormontage die Nadelschutzkappe 12 aufgesteckt werden, wobei eine Anzahl von innenseitig an der Nadelschutzkappe 12 angeordnete Rastnasen 90 in die Nut 88 eingreifen. Der Grundkörper 20´ und die aufgesteckte Nadelschutzkappe 12 bilden somit das in FIG. 14 Im Längsschnitt (FIG. 14a) und in seitlicher Ansicht (FIG. 14b) gezeigte Ensemble, das gemeinsam auf den Spritzenkörper 2´ aufgesteckt werden kann.

Wie der Darstellung in FIG. 14 zudem entnehmbar ist, ist gemäß einem Aspekt der Erfindung auch in dieser Variante die Nadelschutzkappe 12 als Originalitätsverschluss ausgeführt. Dazu umfasst sie den eigentlichen Kappenkörper, an den in der Art eines Siegels abreißbar ein Sicherungsring 49 angeformt ist. Beim Öffnen der Spritze, also beim Entfernen der Nadelschutzkappe 12 vom Nadelkopf 6´, wird der Sicherungsring 49 abgerissen, und er verbleibt auf dem Nadelhalter 6´. Damit wird für den Benutzer eindeutig erkennbar, dass die Spritze 1 bereits benutzt und der Wirkstoffcontainer "angebrochen" wurde.

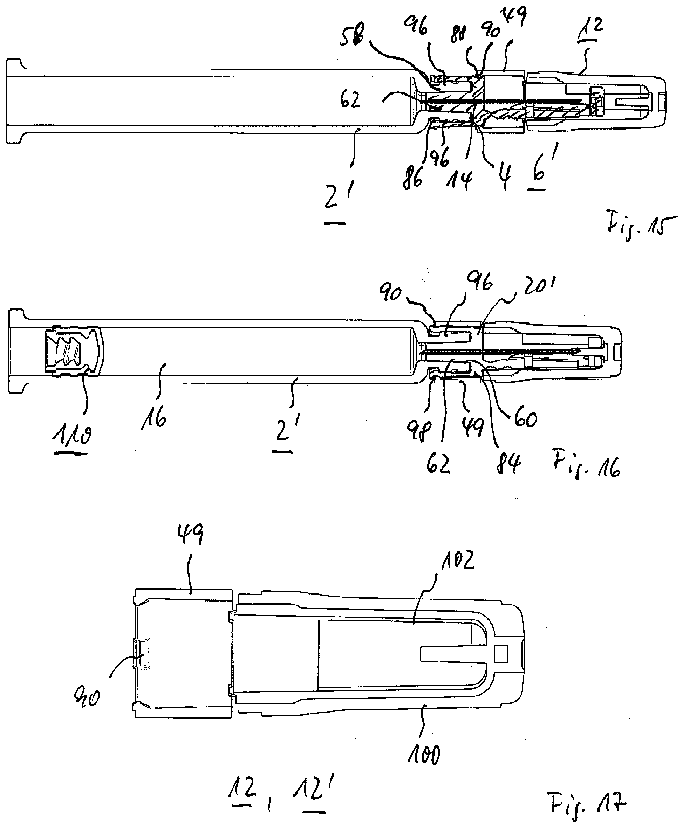

Beim Anbringen der Aufprellkappe 84 kann diese somit auf den mit einer geeignet angepassten Außenwulst 96 versehenen endseitigen Mündungsbereich des Spritzenkörpers 2´, wobei die Rastelemente 86 zunächst durch die Außenwulst 96 nach außen gebogen werden und anschließend, nach weiterem Aufschieben, die Außenwulst 96 hintergreifen und in der Art einer Schnappverbindung mit dieser verrasten. Der solchermaßen mit der teilweise aufgeschobenen Aufprellkappe 84 versehene Spritzenkörper 2´ ist in FIG. 15 im Längsschnitt gezeigt. Deutlich ist dabei erkennbar, dass auch in dieser Variante gemäß einem Aspekt der Erfindung der in dieser Variante mit der Außenwulst 96 versehene endseitige Verbindungszapfen 58 des Spritzenkörpers 2´ ebenfalls mittig den sich zum distalen Ende 4 hin aufweitenden Aufnahmekanal 60 für den zugeordneten, geometrisch an diesen angepassten, das Nadellager 44 tragenden Steckzapfen 62 des Nadelhalters 6´ aufweist. Beim Einstecken des Steckzapfens 62 wird dieser zunehmend in den Aufnahmekanal 60 eingeschoben, bis aufgrund der konischen Ausgestaltung des Steckzapfens 62 einerseits und daran angepasst des Aufnahmekanals 60 andererseits ein flächiger Kontakt zwischen diesen Komponenten entsteht und zudem die Rastelemente 86 mit der Außenwulst 96 verrasten. Dieser Zustand, mit dem gemäß einem Aspekt der Erfindung eine besonders dichte und formbedingt auch mechanisch besonders stabile Verbindung des Nadelkopfs 6´ mit dem Spritzenkörper 2´ erreicht ist, ist in FIG. 15 gezeigt.

Des Weiteren ist gemäß einem Aspekt der Erfindung der die Aufprellkappe 84 bildende Endbereich des Grundkörpers 20´ mit einer im unteren oder Schürzenbereich der Aufprellkappe 84 angeordneten weiteren Nut 98 versehen. Zum endgültigen Verschließen des Spritzenkörpers 2´ kann somit, wie dies der Darstellung im Längsschnitt gem. FIG. 16 entnehmbar ist, die Nadelschutzkappe 12 insgesamt und somit auch mit ihrem Sicherungsring 49 noch weiter auf die Aufprellkappe 84 aufgeschoben werden, bis die Rastnasen 90 nunmehr in die Nut 98 eingreifen. Damit ist der Nadelhalter 12´ rastend auf dem Nadelkopf 6´ fixiert, und der Sicherungsring 49 umschließt den Schürzenbereich der Aufprellkappe 84. Damit wird die rastende Verbindung der die Außenwulst 96 hintergreifenden Rastelemente 86 fixiert, da diese nun nicht mehr nach außen ausweichen können und die Verrastung nunmehr festgelegt ist.

Gemäß einem als eigenständig erfinderisch angesehenen Aspekt ist die Nadelschutzkappe 12, 12´ zweikomponentig ausgeführt. Dies ist der Darstellung der Nadelschutzkappe 12, 12´ im Längsschnitt in FIG. 17 gut entnehmbar. Die Nadelschutzkappe 12, 12´ umfasst gemäß einem Aspekt der Erfindung dabei einen Außenkörper 100 aus einem vergleichsweise harten Kunststoffmaterial, im Ausführungsbeispiel Polypropylen (PP). Dieser dient der Form- und Strukturbildung der Nadelschutzkappe 12, 12´ und bildet auch den Sicherungsring 49 einschließlich der daran innenseitig angeformten Rastnasen 90 aus. Innenseitig ist der Außenkörper 100 gemäß einem Aspekt der Erfindung mit einer Auskleidung 102 versehen, die aus einem vergleichsweise weichen Kunststoff, im Ausführungsbeispiel TPE, gebildet ist. Die Auskleidung 102 dient dabei in besonderem Maße dem Nadelschutz, also dem Schutz der Hohlnadel 14, solange diese in der Nadelschutzkappe 12,12´ geführt oder von dieser umschlossen ist. Des Weiteren kann die Auskleidung 102 gemäß einem Aspekt der Erfindung hinsichtlich ihrer Positionierung und geometrischen Ausführung, insbesondere ihrer Dicke und/oder räumlichen Verteilung innerhalb des Außenkörpers 100, als Dichtelement für das distale, an sich frei liegende Ende 36 der Hohlnadel 14 ausgeführt sein. Die Ausgestaltung kann dabei gemäß einem Aspekt der Erfindung beispielsweise derart vorgesehen sein, dass das distale Ende 36 der Hohlnadel 14 bei auf den Nadelkopf 6 aufgesetzter Nadelkappe 12, 12´ in gewissem oder geringem Maße in die Auskleidung 102 einsticht, so dass es durch das Material der Auskleidung 102 dichtend verschlossen wird.

Innerhalb des den jeweiligen Spritzenkörper 2, 2´ bildenden Hohlkörpers 16 ist ein in Längsrichtung verschiebbarer Kolben oder Stopfen 110 vorgesehen, wie dies für die beiden gezeigten Varianten beispielsweise aus der jeweiligen Darstellung im Längsschnitt gem. FIG. 11 bzw. FIG. 16 erkennbar ist. Der Stopfen 110 ist dabei in an sich durchaus üblicher Ausgestaltung über das jeweils am proximalen Ende des Spritzenkörpers 2, 2´ angeordnete Betätigungselement 10, beispielsweise einen Betätigungsstößel oder auch ein Element mit automatisiertem Antrieb, innerhalb des Hohlkörpers 16 verschiebbar, so dass beispielsweise der im Hohlkörper 16 vorgehaltene Wirkstoff über die Hohlnadel 14 ausgetragen und somit appliziert werden kann. Der Stopfen 110 ist dabei derart dimensioniert, dass er dichtend an der Innenwand des Hohlkörpers 16 anliegt; im Längsbereich des Innenraums des Hohlkörpers 16 zwischen dem distalen Ende 4 und dem Stopfen 110 wird somit das Reservoir für den Wirkstoff ausgebildet, in dem dieser vorgehalten werden kann. Bei angebrachtem Nadelkopf 6, 6´, bei dem die Hohlnadel 14 mit ihrem proximalen Ende in dieses Reservoirvolumen hineinragt, kann der Stopfen 110 innerhalb des Hohlkörpers 16 in Richtung zum distalen Ende 4 hin verschoben werden, so dass der Wirkstoff durch die Hohlnadel 14 aus dem Innenraum herausgedrückt wird.

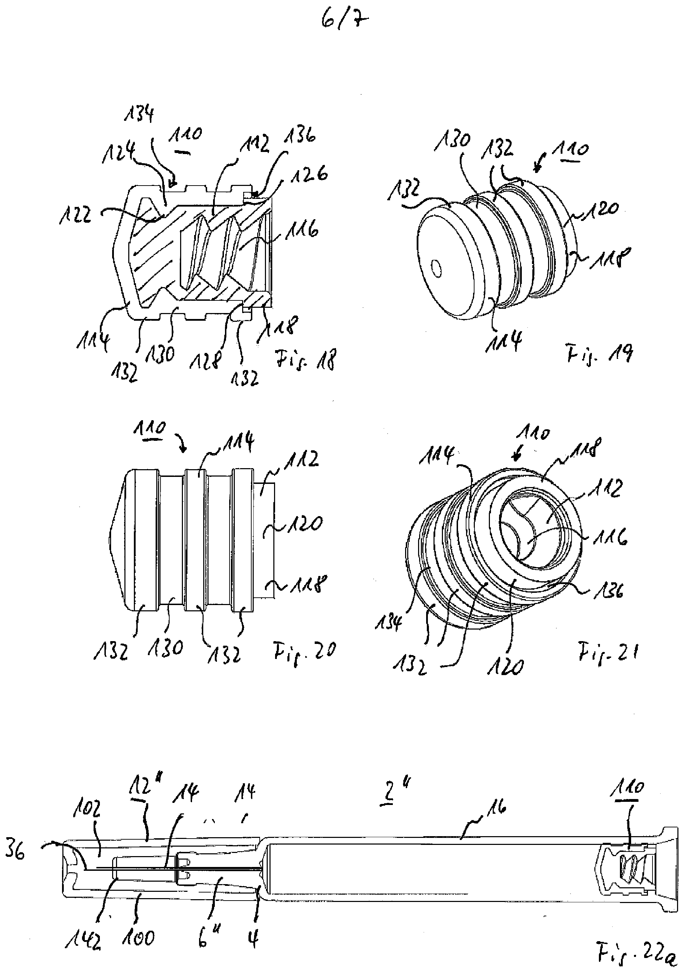

Um auch höchsten Ansprüchen hinsichtlich Dichtheit einerseits und Gleiteigenschaften innerhalb des Hohlkörpers 16, beispielsweise hinsichtlich der Losbrechkraft bei Betätigung des Stopfens 110, andererseits Genüge zu tun, ist der Stopfen 110 in als eigenständig erfinderisch angesehener Ausgestaltung mehrkomponentig, insbesondere zweikomponentig, ausgeführt. Details zur Ausgestaltung des Stopfens 110 sind den Darstellungen im Längsschnitt in FIG. 18, in perspektivischer Ansicht in FIG. 19, in seitlicher Ansicht in FIG. 20 und in weiterer perspektivischer Ansicht "von hinten" in FIG. 21 entnehmbar.

Der gemäß einem Aspekt der Erfindung zweikomponentig oder als "2K-Stopfen" ausgeführte Stopfen 110 umfasst einen zentralen Stopfenkörper 112 aus einem vergleichsweise harten Kunststoff, im Ausführungsbeispiel und gemäß einem Aspekt der Erfindung aus Polypropylen (PP), der von einem aus im Vergleich dazu weicheren Kunststoffmaterial, im Ausführungsbeispiel und gemäß einem Aspekt der Erfindung TPE, gebildeten Dichtmantel 114 umgeben ist. Der zentrale Stopfenkörper 112 dient dabei im Wesentlichen zur Form- und Strukturstabilität und zur Weiterleitung der eingeleiteten Kräfte, insbesondere in Längsrichtung bei der Verschiebung des Stopfens 110 innerhalb des Spritzenkörpers 2, 2´. Dazu kann der zentrale Stopfenkörper 112 innenseitig mit einem Aufnahmekanal 116, beispielsweise mit einem Gewinde, versehen sein, über den eine Verbindung mit einem externen Betätigungsmittel, beispielsweise dem Betätigungselement 10 oder dem Betätigungsstößel der Spritze 1, ermöglicht ist.

Diese gemäß einem Aspekt der Erfindung vorgesehene Bauweise, also die Bereitstellung des inneren, vergleichsweise härteren Stopfenkörpers 112 mit einer Ummantelung durch den vergleichsweise weicheren Dichtmantel 114, mit dem im Aufnahmekanal 116 vorgesehen stabilen Gewinde wird als besonders vorteilhaft angesehen. Durch diese Bauweise kann der Stopfenkörper 112 und mit diesem der Stopfen 110 insgesamt fest mit dem Betätigungselement 10 der Spritze 1 verbunden werden. Dadurch können axiale Verschiebungen des Stopfens 110 innerhalb des Hohlkörpers 16 sowohl in "Vorwärts- " als auch in "Rückwärtsrichtung" erfolgen, d. h. es können wahl- und bedarfsweise Schub- oder auch Zugwirkungen auf den Stopfen 110 ausgeübt werden. Damit sind einerseits Anwendungen wie z. B. Aspirationstests und/oder Rekonstitutionen von Medikamenten in einer solchen Spritze ermöglicht. Andererseits können auch vergleichsweise hohe Haltekräfte in einem solchen System toleriert werden, die mit herkömmlichen Stopfenverbindungen nicht möglich wären. Des Weiteren ist üblicherweise beim Einschrauben der Kolbenstange in ein entsprechendes Gewinde eines reinen TPE- oder Gummistopfens ein erhöhter Anpressdruck in Kauf zu nehmen, so dass sich die Haft- oder Gleitreibung entsprechend erhöht. Dieser Nachteil ist durch die gemäß einem Aspekt der Erfindung nunmehr vorgesehene Ausgestaltung des Stopfens 110 nicht mehr in Kauf zu nehmen.

Der Dichtmantel 114 umgibt den zentralen Stopfenkörper 112 gemäß einem Aspekt der Erfindung in Längsrichtung gesehen nahezu vollständig, wobei lediglich im proximalen Anschlussbereich 118 des Stopfenkörpers 112 ein endseitiger Flansch 120 vom Dichtmantel 114 unbedeckt bleibt. Der zentrale Stopfenkörper 112 ist dabei für eine formschlüssige Fixierung des Dichtmantels 114 mit einer umlaufenden Haltenut 122 versehen, in die eine Innenlippe 124 des Dichtmantels 114 eingreift. Des Weiteren weist der zentrale Stopfenkörper 112 im Bereich des endseitigen Flanschs 120 eine umlaufende Kante 126 auf, auf der eine korrespondierende Innenkante 128 des Dichtmantels 114 aufsitzt.

In einer als eigenständig erfinderisch angesehenen Ausführung ist der Stopfen 110 für eine besonders hohe Dichtwirkung innerhalb des jeweiligen Spritzenkörpers 2, 2´ ausgelegt. Dabei wird gemäß einem Aspekt der Erfindung gezielt die relativ gute Verformbarkeit des vergleichsweise weichen Dichtmantels 114 mit einer geeigneten Geometriewahl und Formgebung kombiniert. Dazu weist der Dichtmantel 114 einen im Wesentlichen zylindrischen Außenmantel 130 auf, der mit einer Mehrzahl von in Richtung der Zylinderachse gesehen beabstandet zueinander positionierten umlaufenden Dichtrippen 132 versehen ist. Im Bereich der Dichtrippen 132 kann der Stopfen 110 gemäß einem Aspekt der Erfindung mit einem gewissen Übermaß ausgeführt sein, d. h. einen geringfügig größeren Durchmesser aufweisen als der Innendurchmesser des jeweiligen Spritzenkörpers 2, 2´. Aufgrund der Verformbarkeit des den Dichtmantel 114 bildenden Kunststoffs kann dieser sich somit an die Innenkontur des Spritzenkörpers 2, 2´ anpassen, wobei aufgrund der Formgebung und der vorgesehenen beabstandet voneinander angeordneten Dichtrippen 132 Ausweichräume bereitgestellt sind, in die die verformten Dichtrippen 132 ausweichen können. Somit ist es möglich, einerseits auf zuverlässige Weise eine hohe Dichtwirkung zu erzielen, wobei andererseits die auftretenden Haft- und Gleitreibungskräfte infolge des Kontakts mit der Innenwand des jeweiligen Spritzenkörpers 2, 2´ ausreichend gering gehalten werden können. Gemäß einem Aspekt der Erfindung ist dabei die Breite der oder jeder Dichtrippe 132 geringer gewählt als der Abstand zwischen zwei benachbarten Dichtrippen 132. Durch diese Ausgestaltung des Stopfens 110 kann somit bei hoher Dichtigkeit eine besonders gute und störungsfreie Verschiebbarkeit des Stopfens 110 im jeweiligen Spritzengehäuse 2, 2´ erreicht werden.

Zusätzlich begünstigt werden diese Effekte durch die gemäß einem Aspekt der Erfindung vorgesehene Positionierung und Ausgestaltung der Haltenut 122 mit korrespondierender Innenlippe 124, die im Schnitt gesehen jeweils eine Art V-förmige Kontur aufweisen. Damit wird unter Nutzung der Verformbarkeit des relativ weichen Materials des Dichtmantels 114 eine Verformungszone 134 geschaffen, in die hinein Material aus der in Vorschubrichtung des Stopfens 110 gesehen vordersten Dichtrippe 132 ausweichen kann. Damit wird der Reibwiderstand des im Spritzenkörper 2, 2´ gleitenden Stopfens 110 gemäß einem Aspekt der Erfindung noch weiter verringert.

Zur weiteren Begünstigung des Reibverhaltens des Stopfens 110 weist gemäß einem Aspekt der Erfindung der Dichtmantel 114 im Bereich des endseitigen Flanschs 120 und seiner Innenkante 128 einen im Vergleich zum Außendurchmesser des zentralen Stopfenkörpers 112 geringfügig vergrößerten Innendurchmesser auf, so dass sich im montierten Zustand endseitig ein Spalt in der Art einer umlaufenden Nut 136 zwischen diesen Komponenten ergibt. Durch diese umlaufende Nut 136 wird eine weitere Verformungszone bereitgestellt, in die bei einer Reibbelastung Material der in Vorschubrichtung des Stopfens 110 gesehen letzten Dichtrippe 132 ausweichen kann.

Gemäß einem Aspekt der Erfindung ist der Stopfen 110 durch ein 2K-Spritzgussverfahren hergestellt.

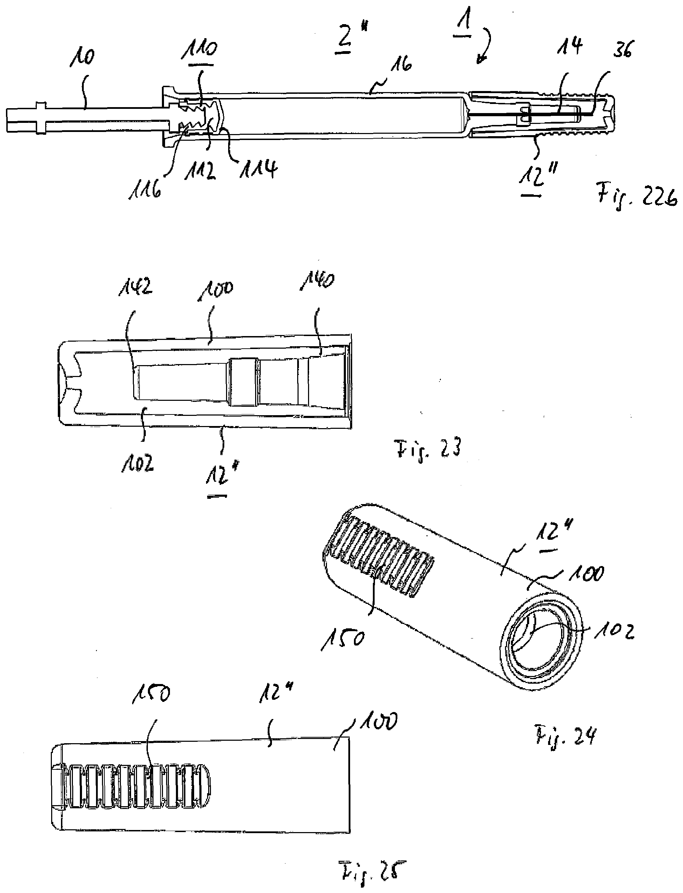

Ein weiterer alternativer Spritzenkörper 2´´, bei dem die genannten als erfinderisch angesehenen Aspekte zum Einsatz kommen, ist in FIG. 22a im Längsschnitt gezeigt. In dieser als eigenständig erfinderisch angesehenen Ausführungsform ist der Spritzenkörper 2´´ besonders einfach ausgeführt und insbesondere für eine silikonfreie Ausgestaltung besonders geeignet. Dieser Spritzenkörper 2´´ umfasst ebenfalls einen Hohlkörper 16, in dem ein zweikomponentiger Stopfen 110 der vorstehend beschriebenen Art geführt ist. An seinem distalen Ende 4 weist der Hohlkörper 16 in dieser Ausführungsform einen fest installierten, beispielsweise angeformten, Nadelkopf 6´´ auf, in dem die Hohlnadel 14 gelagert ist. In FIG. 22b ist der Spritzenkörper 2´´ mit angebrachtem Betätigungselement 10, in der gezeigten Ausführungsform als Betätigungsstößel ausgeführt, dargestellt. Die solchermaßen gebildete Spritze 1, bei der der Betätigungsstössel mit seinem distalen Ende 138 in das im Aufnahmekanal 116 des Stopfens 110 vorgesehene Gewinde eingeschraubt ist, wird ausdrücklich als eigenständig erfinderisch angesehen.

In der gezeigten Ausführungsform ist gemäß einem Aspekt der Erfindung ebenfalls eine Nadelschutzkappe 12´´ in zweikomponentiger (oder 2K-) Ausführung wie vorstehend beschrieben vorgesehen. In dieser Ausführung ist gemäß einem Aspekt der Erfindung, wie insbesondere der vergrößerten Darstellung im Längsschnitt gem. FIG. 23 entnehmbar, die innerhalb des Außenkörpers 100 angeordnete Auskleidung 102 in einem zur Aufnahme des vorderen Bereichs des Nadelkopfs 6´´ vorgesehenen Aufnahmebereich 140 mit ihrer Innenkontur an die Außenkontur des Nadelkopfs 6´´ angepasst. Auf diese Weise kann der Nadelkopf 6´´ sicher in den Aufnahmebereich 140 eingesteckt werden, wobei die Nadelschutzkappe 12´´ gerade aufgrund der guten Verformbarkeit der vergleichsweise weichen Auskleidung 102 besonders gut und wirksam in Position gehalten wird. In ihrem frontalen Endbereich 142 ist die Auskleidung 102 gemäß einem weiteren Aspekt der Erfindung hingegen besonders dick ausgeführt und in ihrer Geometrie derart ausgeführt, dass bei aufgesetzter Nadelschutzkappe 12´´ die Hohlnadel 14 mit ihrer Spitze in den frontalen Endbereich 142 der Auskleidung 102 eindringt und somit dort fixiert und abgedichtet wird.

Die Nadelschutzkappe 12´´ ist vergrößert in perspektivischer Ansicht in FIG. 24 und in seitlicher Ansicht in FIG. 25 gezeigt. Deutlich erkennbar ist dabei, dass der Außenkörper 100 außenseitig mit Griffflächen 150 zur erleichterten Handhabung versehen ist.

### Bezugszeichenliste

- 1: Medizinische Spritze
- 2,2´: Spritzenkörper
- 4: distales Ende
- 6, 6´: Nadelkopf
- 8: proximales Ende
- 10: Betätigungselement
- 12, 12´: Nadelschutzkappe
- 14: Hohlnadel
- 16: Hohlkörper
- 20: Grundkörper
- 26: Nadelschutzhülle
- 28: Gelenk
- 30: Schutzkörper
- 32: Aufnahmerinne
- 34: Deckplatte
- 36: freies Ende
- 38: Betätigungselement
- 40: Anschlussbereich
- 42: Luer-Gewinde
- 44: Nadellager
- 46: Rippe
- 48: Rastnase
- 49: Sicherungsring
- 50: Luer-Innengewinde
- 52: Anschlussstutzen
- 54: Verbindungssystem
- 56: Gewinderaum
- 58: Verbindungszapfen
- 60: Aufnahmekanal
- 62: Steckzapfen
- 64: Vertiefung
- 66: Kragen
- 68: Rastnocken
- 84: Aufprellkappe
- 86: Rastelement
- 88: Nut
- 90: Rastnase
- 96: Außenwulst
- 98: Nut
- 100: Außenkörper
- 102: Auskleidung
- 110: Stopfen
- 112: Stopfenkörper
- 114: Dichtmantel
- 116: Aufnahmekanal
- 118: Anschlussbereich
- 120: Flansch
- 122: Haltenut
- 124: Innenlippe
- 126: Kante
- 128: Innenkante
- 130: Außenmantel
- 132: Dichtrippe
- 134: Verformungszone
- 136: Nut
- 138: Ende
- 140: Aufnahmebereich
- 142: Endbereich
- 150: Griffflächen

## Patentansprüche

1. Nadelschutzkappe (12, 12´, 12´´) für eine medizinische Spritze (1), mit einem Außenkörper (100) aus einem ersten, vergleichsweise harten Kunststoff, der innenseitig mit einer Auskleidung (102) aus einem zweiten, im Vergleich zum ersten Kunststoff weicheren Kunststoff versehen ist.

2. Nadelschutzkappe (12, 12´, 12´´), nach Anspruch 1, erhalten durch ein 2K-Spritzgussverfahren.

3. Nadelschutzkappe (12, 12´, 12´´) nach Anspruch 1 oder 2, deren Außenkörper (100) aus PP gebildet ist.

4. Nadelschutzkappe (12, 12´, 12´´) nach einem der vorigen Ansprüche, deren Auskleidung (102) aus TPE ausgeführt ist.

5. Medizinische Spritze (1) mit einem Spritzenkörper (2, 2´, 2´´), an dessen distalem Ende (4) eine in einem Nadelkopf (6) angeordnete Hohlnadel (18) angeordnet ist, und mit einer Nadelschutzkappe (12, 12´, 12´´) nach einem der vorigen Ansprüche.

6. Spritze (1) nach Anspruch 5, bei der die Auskleidung (102) der Nadelschutzkappe (12, 12´, 12´´) in deren frontalem Endbereich (142) derart verdickt ausgeführt und positioniert ist, dass bei auf den Spritzenkörper (2, 2´, 2´´) aufgesetzter Nadelschutzkappe (12, 12´, 12´´) die Hohlnadel (14) mit ihrer Spitze (36) in den frontalen Endbereich (142) der Auskleidung (102) eindringt und somit dort fixiert und abgedichtet wird.

7. Spritze (1) nach Anspruch 5 oder 6, bei der die Auskleidung (102) der Nadelschutzkappe (12, 12´, 12´´) in einem zur Aufnahme des vorderen Bereichs des Nadelkopfs (6) vorgesehenen Aufnahmebereich (140) mit ihrer Innenkontur an die Außenkontur des Nadelkopfs (6) angepasst ist.
